# EUROPÄISCHE PATENTSCHRIFT

(11) **EP 1 147 433 B1**
(45) Veröffentlichungstag und Bekanntmachung des Hinweises auf die Patenterteilung: **20.11.2002**
(21) Anmeldenummer: 99964574.0
(22) Anmeldetag: 14.12.1999
(51) Int. Cl.: G01S 7/52, G01S 15/89, A61B 8/14

(54) **VERFAHREN ZUR BEWEGUNGSKOMPENSATION BEI ULTRASCHALLAUFNAHMEN EINES OBJEKTS**
METHOD FOR COMPENSATING MOTION IN ULTRASOUND IMAGES OF AN OBJECT
PROCEDE PERMETTANT DE COMPENSER LE MOUVEMENT LORS DE PRISES DE VUES PAR ULTRASONS D'UN OBJET

(30) Priorität: 28.01.1999 DE 19903332
(43) Veröffentlichungstag der Anmeldung: 24.10.2001
(73) Patentinhaber: TomTec Imaging Systems GmbH, 85716 Unterschleissheim (DE)
(72) Erfinder: WALDINGER, Johannes, D-85521 Ottobrunn (DE); DUNKHASE, Kim, Marlies, D-25451 Quickborn (DE); MUMM, Bernhard, D-82291 Mammendorf (DE); SCHRECKENBERG, Marcus, D-85354 Freising (DE)
(74) Vertreter: Müller, Frank Peter, Dipl.-Ing.
(86) Internationale Anmeldenummer: EP9909935
(87) Internationale Veröffentlichungsnummer: WO00045193

(56) Entgegenhaltungen:
- EP-A- 0 514 584
- EP-A- 0 647 457
- EP-A- 0 736 284
- EP-A- 0 961 135
- WO-A-97/32277
- WO-A-99/05542
- US-A- 5 538 004

## Beschreibung

Die Erfindung betrifft ein Verfahren zur Bewegungskompensation bei Ultraschallaufnahmen eines Objekts, insbesondere zur Bewegungskompensation bei Ultraschallaufnahmen und der Darstellung dreidimensionaler Ultraschallbilder in Echtzeit nach dem Oberbegriff des Anspruchs 1 sowie eine Verwendung dieses Verfahrens nach den Ansprüchen 14 und 15.

Es sind Verfahren zur Aufnahme von Ultraschallbildern eines Objekts bekannt (DE 197 30 938 bzw. DE 197 32 125 oder DE 198 38 140), bei denen ein Ultraschallsender Ultraschallwellen auf ein Objekt abstrahlt, während ein Ultraschallempfänger die von dem Objekt reflektierten Ultraschallwellen empfängt. Sowohl der Ultraschallsender als auch der Ultraschallempfänger sind Bestandteile eines Ultraschallaufnahmesystems, die beispielsweise in einem Ultraschallkopf gemeinsam realisiert sein können. Zur Aufnahme des Objekts wird zumindest ein Teil des Ultraschallaufnahmesystems entlang dem Objekt verfahren bzw. relativ zum Objekt rotiert, während einzelne Bildteilbereiche des Objekts aufgenommen werden. Diese Bildteilbereiche entsprechen im Normalfall einem zeilenweisen Scan-Vorgang, der das Objekt zeilenweise längs einer Aufnahmerichtung, in der das Ultraschallaufnahmesystem bzw. zumindest ein Teil des Ultraschallaufnahmesystems verfahren wird, aufnimmt.

Die im Ultraschall-Aufnahmesystem erzeugten Bilder lassen sich bei den bekannten Geräten digital oder über einen Videoausgang in ein Nachverarbeitungsgerät bzw. in ein Datenverarbeitungssystem übernehmen. Dort können die Bilder gespeichert oder auch direkt nachverarbeitet werden. Gegebenenfalls werden aber auch direkt die einzelnen Bildteilbereiche in das Nachverarbeitungsgerät übertragen, um dort entsprechend zu einem Ultraschallbild zusammengesetzt zu werden. Durch den Scan-Vorgang wird das zu untersuchende Objekt zeilenweise aufgenommen, das heißt, es werden einzelne zueinander parallele "Schichten" bzw. zueinander rotationssymmetrische "Scheiben" des Objekts mit Ultraschlallwellen beaufschlagt und die entsprechenden reflektierten Ultraschallwellen in dem Ultraschallaufnahmesystem empfangen. Die empfangenen Ultraschallwellen werden in dem Datenverarbeitungssystem verarbeitet, so daß sich an einem Anzeigegerät ein Grauwertbild ergibt, wobei die einzelnen Grauwerte entsprechend stärker oder schwächer reflektierten Ultraschallwellen entsprechen. Diese Grauwerte liefern Informationen über die einzelnen Schichten bzw. Scheiben des Objekts, das heißt darüber, an welchen Stellen das Objekt beispielsweise Hohlräume oder höher verdichtete Materialbereiche aufweist.

Die einzelnen Schichten bzw. Scheiben des Objekts, das heißt die einzelnen Zeilenaufnahmen des Ultraschall-Scan-Vorganges werden in dem Datenverarbeitungssystem "übereinandergeschichtet", um dann am Anzeigegerät eine dreidimensionale Darstellung des Objekts zu erhalten. Die unterschiedlichen Abstände verschiedener Bereiche einer Schicht, das heißt eines Bildteilbereichs, das heißt die Lage von Hohlräumen oder stärker verdichteten Materialbereichen des Objekts relativ zu dem Ultraschallgerät erhält man durch die Auswertung der Grauwertinformationen einer jeden Schicht.

Die Ultraschallverfahren benützen eine vordefinierte oder eine zu errechnende Grauwertstufe (Threshold), um im Bild Konturen zu finden. Die Kontureninformationen werden dann in einem Bild gespeichert und ergeben nach Auswertung der Entfernungen zwischen dem Ultraschallaufnahmesystem und den Konturen des zu untersuchenden Objekts einen dreidimensionalen Bildeindruck.

Diese bekannten Ultraschallverfahren eignen sich beispielsweise zur Untersuchung eines Fötus innerhalb der Bauchhöhle der Mutter oder zur Detektion von Nierensteinen innerhalb des menschlichen Körpers. Insbesondere werden diese Ultraschallverfahren zur Aufnahme des Herzens eines Lebewesens verwandt, um bereits frühzeitig Herzfehler feststellen zu können. Zur Aufnahme des zu untersuchenden Objekts wird das Ultraschallgerät mittels eines Schallmediums, wie beispielsweise Gel, Öl oder Wasser mit der Hautoberfläche des Körpers verbunden und entlang einer gewünschten Aufnahmerichtung bewegt bzw. rotiert, während in bestimmten Zeit- oder räumlichen Abständen Ultraschallbilder aufgenommen werden. Diese einzelnen Bildteilbereiche, das heißt "Schnitte" durch das zu untersuchende Objekt, werden dann in dem entsprechenden Nachverarbeitungsgerät wieder zusammengesetzt, um dort ein dreidimensionales Bild rekonstruieren zu können. Dieses dreidimensionale Bild kann dann wiederum durch berechnete Schnittbilder bestimmte interessierende Bereiche des Objekts darstellen.

In der klinischen Praxis eingesetzte Ultraschallverfahren zur räumlichen Aufnahme von menschlichen Organen arbeiten derzeit tomographisch, das heißt, das Volumen wird aus den einzelnen aufgenommenen Schichtebenen bzw. Scheiben zusammengesetzt. Bei der transösophagealen Echokardiographie wird beispielsweise dem Patienten durch die Speiseröhre eine schwenkbare endoskopische Sonde eingeführt. Der Ultraschallsensor ist als sogenanntes "phase array" in der Spitze der Sonde integriert. Durch den darauffolgenden Aufnahmeprozeß wird beispielsweise das Herz eines Patienten schrittweise aufgenommen und später am Nachverarbeitungsgerät rekonstruiert und dargestellt.

Bei der dreidimensionalen oder der dynamischen dreidimensionalen, das heißt vierdimensionalen Bildaufnahme von tomographischen Schnitten ist es jedoch nicht immer möglich, das gesamte Volumen, das heißt einen kompletten, dreidimensionalen Datensatz in Echtzeit zu erfassen. Dies liegt insbesondere daran, daß die Zeit für die Aufnahme eines kompletten dreidimensionalen Datensatzes mehrere Minuten in Anspruch nehmen kann. Zusätzlich müssen die einzelnen Aufnahmesequenzen mit den periodischen Bewegungen der zu erfassenden Objekte synchronisiert werden. Dies kann beispielsweise der Herzrhythmus sein oder die Atemlage, die anhand des EKG bzw. des Pulses des Patienten festgestellt werden können.

Bei der Synchronisierung der Aufnahmesequenzen mit den periodischen Bewegungen des zu erfassenden Objekts ergeben sich durch notwendige Toleranzfenster bzw. durch zufällige Abweichungen von der periodischen Bewegung sogenannte Lageartefakte. Diese Lageartefakte sind Abweichungen der Lage der Objekte von der zu erwartenden Lage. Bewegt sich der Patient während der Aufnahmesequenz, so verschiebt sich der zu diesem Zeitpunkt aufgenommene Bildteilbereich relativ zu den anderen aufgenommenen Bildteilbereichen in einer Art und Weise, die nicht erfaßt werden kann. Zusätzlich können sich während der Untersuchung des Objekts weitere relative Verschiebungen oder Verdrehungen zwischen dem Ultraschallaufnahmesystem und dem Objekt auftreten, die das System selbst nicht erfassen kann, so daß dann bei der Rekonstruktion des dreidimensionalen oder vierdimensionalen Ultraschallbildes Artefakte entstehen, die zu quantitativen Fehlern führen.

Herkömmliche Verfahren versuchen durch eine geeignete Wahl von Parametern für die entsprechenden Bewegungsabläufe ein Optimum zwischen der Aufnahmezeit und der Bildung von Artefakten zu erreichen. Diese Verfahren benützen entweder eine sehr hohe Aufnahmegeschwindigkeit, so daß entsprechend relative Lagefehler bzw. Bewegungen des Objekts bzw. relative Bewegungen zwischen dem Objekt und zwischen dem Ultraschallaufnahmesystem geringeren Einfluß auf die entsprechende Bildqualität haben. Diese Verfahren leiden jedoch unter dem Nachteil, daß keine qualitativ hochwertigen Bilder entstehen. Eine Lage- und/oder Bewegungskorrektur in allen sechs räumlichen Freiheitsgraden wird derzeit nicht durchgeführt.

WO 97/32277 offenbart ein Verfahren zur Generierung von Ultraschallaufnahmen eines Objekts nach dem Oberbegriff des Patentanspruchs 1.

Der Erfindung liegt daher die Aufgabe zugrunde, herkömmliche Verfahren zur Bewegungskompensation bei Ultraschallaufnahmen eines Objekts insofern zu verbessern, daß eine Korrektur der aufgenommenen Bildteilbereiche hinsichtlich der relativen Bewegungen ermöglicht wird. Die entsprechenden Bewegungen sollen detektiert und in die Darstelllung des Ultraschallbildes einfließen, so daß ein korrigiertes Ultraschallbild mit nur geringen bzw. keinen Lageartefakten realisierbar ist.

Die Erfindung löst die genannte Aufgabe durch die kennzeichnenden Merkmale des Patentanspruchs 1. Vorteilhafte Ausgestaltungen der Erfindung sind in den Unteransprüchen beschrieben und beansprucht.

Das erfindungsgemäße Verfahren zur Bewegungskompensation bei Ultraschallaufnahmen eines Objekts nimmt einzelne Bildteilbereiche des Objekts mittels eines Ultraschallaufnahmesystems auf. Dabei wird mindestens ein Teil des Ultraschallaufnahmesystems zur Aufnahme der einzelnen Bildteilbereiche relativ zum Objekt verfahren und/oder teilweise rotiert, so daß dann die einzelnen Bildteilbereiche zur Erzeugung eines Ultraschallbildes in einem Datenverarbeitungsgerät zusammengesetzt werden können. Die relativen Bewegungen zwischen dem Objekt und dem Ultraschallaufnahmesystem bzw. die zufälligen oder auch periodischen Bewegungen des Objekts selbst werden entweder mittels eines Detektors erfaßt oder mittels eines Modellbildes ermittelt und den einzelnen Bildteilbereichen zugeordnet, so daß dadurch ein korrigiertes Ultraschallbild erzeugt werden kann.

Zu diesem Zweck wird das Objekt bzw. das Ultraschallaufnahmesystem mit einem Detektor verbunden, der die Bewegungen des Objekts bzw. des Ultraschallaufnahmesystems erfaßt. Durch die Erfassung der drei translatorischen und der drei rotatorischen Freiheitsgrade der Bewegung des Objekts bzw. des Ultraschallaufnahmesystems durch den Detektor können die Abweichungen der einzelnen aufgenommenen Bildteilbereiche während des Aufnahmevorgangs von der realen Kontur des Objekts ermittelt werden, so daß dann bei der späteren Zusammensetzung der einzelnen Bildteilbereiche im Bilddatenverarbeitungsgerät diese Abweichungen berücksichtigt werden können, so daß das Objekt selbst trotz der genannten Relativbewegungen realitätsgetreu nachgebildet und als korrigiertes Ultraschallbild dargestellt werden kann.

Insbesondere werden die relativen Bewegungen zwischen dem Objekt und dem Ultraschallaufnahmesystem bzw. die zufälligen Bewegungen des Objekts selbst, die auch relativ zu den periodischen Bewegungen des Objekts existieren können, mittels des Detektors während der Aufnahme der Bildteilbereiche erfaßt, so daß die Darstellung des korrigierten Ultraschallbildes in Echtzeit, das heißt während der Aufnahme des Objekts, realisiert werden kann. Durch die Erfassung sämtlicher sechs Freiheitsgrade der Bewegungen des Ultraschallaufnahmesystems und/oder des Objekts, die pro aufgenommenem Bildteilbereich diesen entsprechenden Bildteilbereichen zugeordnet werden können, läßt sich ein korrigiertes Ultraschallbild dadurch darstellen, daß die entsprechenden Daten der entsprechenden Positionen des Objekts bzw. des Ultraschallaufnahmesystems mit den Daten der einzelnen Bildteilbereiche kombiniert werden, um so zu einer realitätsgetreuen Nachbildung des aufzunehmenden Objekts zu gelangen.

Zur Ermittlung des korrigierten Ultraschallbildes wird ein aus den aufgenommenen Bildteilbereichen rekonstruiertes Modellbild approximiert bzw. geglättet, so daß sich ein approximiertes, bzw. geglättetes Modellbild ergibt, so daß die relativen Abweichungen zwischen dem rekonstruierten Modellbild und dem approximierten Modellbild ermittelt werden können. Zu diesem Zweck wird das aus den aufgenommenen Bildteilbereichen rekonstruierte Modellbild mittels einer Modelloberfläche, bsp. einem Drahtgittermodell, das vorgegeben ist, approximiert bzw. geglättet, um die durch die relativen oder absoluten Bewegungen entstandenen Lageartefakte zu minimieren. Die relativen Abweichungen werden dabei ebenfalls in den drei translatorischen und den drei rotatorischen Freiheitsgraden ermittelt. Zur Approximation des rekonstruierten Modellbildes eignet sich eine geeignete Fehlernorm zwischen dem Oberflächenmodell und der Oberfläche des rekonstruierten Modellbildes. Durch die Minimierung bsp. des quadratischen Fehlers ergibt sich eine mathematisch genaue Approximation zur Ermittlung eines korrigierten Ultraschallbildes, wenn die zufälligen relativen Bewegungen statistisch normalverteilt sind.

Auf Basis dieses approximierten Modellbildes können dann die relativen Abweichungen der Konturen für jeden einzelnen (aufgenommenen) Bild-Teilbereich gegenüber der Realität in allen sechs translatorischen und rotatorischen Freiheitsgraden geschätzt werden, so daß sich korrigierte Bildteilbereiche ergeben, die sich in einem korrigierten Ultraschallbild darstellen lassen.

Besonders vorteilhaft ist es dabei, nach der Ermittlung der relativen Abweichungen der einzelnen aufgenommenen Bildteilbereiche in Abhängigkeit bestimmter Anforderungen an das korrigierte Ultraschallbild bzw. in Abhängigkeit des Approximationsfehlers, sukzessive weitere Modellbilder zu approximieren, um mittels eines iterativen Verfahrens schließlich zu einem interpolierten Ultraschallbild zu gelangen. Dabei können auch die relativen Abweichungen zwischen den einzelnen aufgenommenen Bildteilbereichen und den entsprechenden Bildteilbereichen des Modellbildes in allen sechs Freiheitsgraden pro Bildteilbereich geschätzt werden, um so nach der Ermittlung des Schätzfehlers diesen iterativ bis zu einem Optimalwert zu reduzieren.

Zusätzlich zu den relativen Bewegungen lassen sich auch die periodischen Bewegungen des Objekts erfassen und den einzelnen aufgenommenen, approximierten oder korrigierten Bildteilbereichen zuordnen, so daß sich ein "dynamisch" korrigiertes Ultraschallbild ergibt, welches entsprechend den periodischen Bewegungen des Objekts dargestellt werden kann, das heißt ein bewegtes korrigiertes Ultraschallbild. Dadurch können bewegte dreidimensionale Ultraschallbilder dargestellt werden, die auch als vierdimensionale Ultraschallbilder bezeichnet werden.

Die Zeitpunkte zur Aufnahme von einzelnen Bildteilbereichen des Objekts lassen sich dann durch die erfaßten Bewegungen steuern bzw. regeln, insbesondere durch die erfaßten periodischen Bewegungen des Objekts. Dadurch eignet sich das Verfahren insbesondere zur Aufnahme eines menschlichen bzw. tierischen Organs, in dem die Zeitpunkte zur Aufnahme der einzelnen Bildteilbereiche anhand von Signalen des Elektrokardiogramms, der Atmung, der Magenbewegung, der Perestaltik der Speiseröhre oder einer Kombination aus diesen Signalen des Lebewesens gesteuert bzw. geregelt werden.

Die einzelnen Bereiche des Objekts, insbesondere unterschiedlich bewegte Bereiche des Objekts, können dann bei der Darstellung farblich gekennzeichnet werden. Die farbliche Kennzeichnung hilft insbesondere dem Arzt, bestimmte Bereiche des Objekts schneller zu erkennen, insbesondere dann, wenn die korrigierten Ultraschallbilder, die aus den Bildteilbereichen gebildet werden, während der Aufnahme des Objekts in Echtzeit verarbeitet bzw. angezeigt werden.

Besonders vorteilhaft läßt sich das Verfahren zur dreidimensionalen Aufnahme eines Organs verwenden, insbesondere zur Aufnahme des Herzens eines Lebewesens unter Berücksichtigung der Bewegung des Herzens zur Darstellung eines bewegten Bildes. Dieses Verfahren eignet sich dann insbesondere zur Verwendung bei der transthorakalen (TTE), transoesophagial (TEE), intravaskulären (IVUS) oder intraductalen (IDUS) Echokardiographie.

Ein besonderes Ausführungsbeispiel der Erfindung wird anhand der folgenden Zeichnungen näher erläutert. Dabei zeigen:
- Fig. 1: ein Ultraschallaufnahmesystem in zwei relativ zueinander unterschiedlichen Aufnahmepositionen;
- Fig. 2: die Darstellung eines Bildteilbereichs mit Lageartefakten;
- Fig. 3: die Darstellung eines Ultraschallaufnahmesystems mit Detektor;
- Fig. 4: die Darstellung eines Oberflächenmodells
- Fig. 5: die Darstellung eines rekonstruierten Modellbildes sowie die Darstellung des oberen Randes (Schnitt der Oberfläche) dieses rekonstruierten Modellbildes;
- Fig. 6: die Darstellung eines approximierten Modellbildes, welches dem rekonstruierten Modellbild nach Fig. 5 überlagert ist, sowie die entsprechende Darstellung der oberen Ränder und
- Fig. 7: die Darstellung des approximierten bzw. geglätteten Modellbildes.

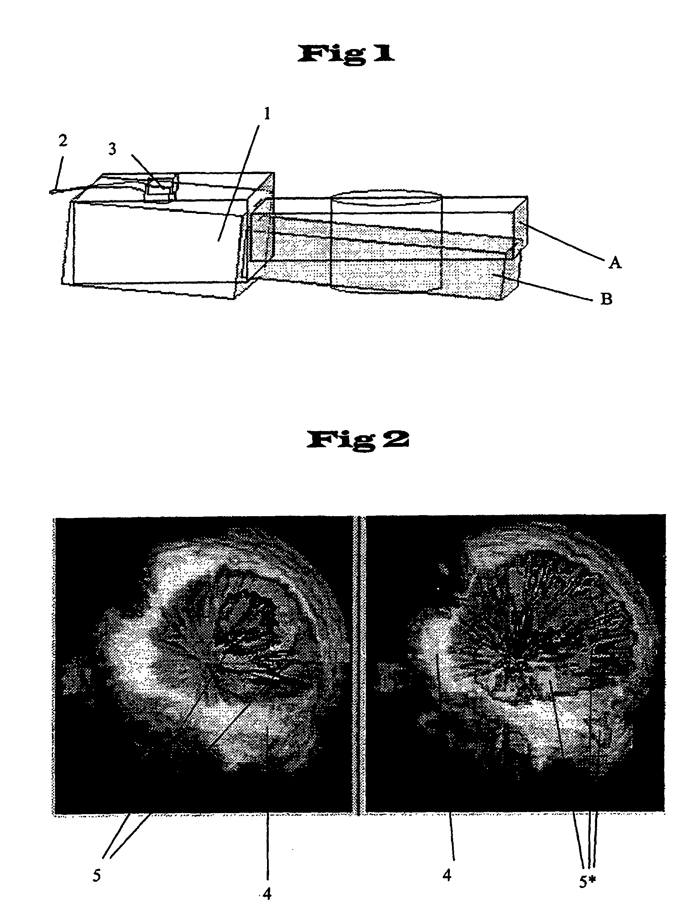

Fig. 1 zeigt ein tomograhisches, dreidimensionales bzw. vierdimensionales Ultraschallaufnahmesystem 1, das während der Aufnahme von Bildteilbereichen mit einem Detektor 3 ausgestattet ist. Dieser Detektor 3 detektiert die drei rotatorischen und die drei translatorischen Freiheitsgrade und übermittelt die entsprechenden Positionen des Ultraschallaufnahmesystems 1 an das Datenverarbeitungssystem (nicht dargestellt) über die Leitung 2. Dargestellt sind zwei unterschiedliche Positionen A und B des Ultraschallaufnahmesystems 1. Beide Positionen A bzw. B werden durch den Detektor 3 erfaßt und an das Datenverarbeitungssystem weitergeleitet. Die entsprechenden Daten des Detektors 3 werden dann zusammen mit den Daten der einzelnen Bildteilbereiche, die durch das Ultraschallaufnahmesystem 1 aufgenommen werden, ausgewertet.

Anhand der durch den Detektor 3 ermittelten Positionen des Ultraschallaufnahmesystems 1 lassen sich die Bewegungen des Ultraschallaufnahmesystems 1 bei der Volumenberechnung des Ultraschallbildes zusammen mit den Daten der einzelnen Bildteilbereiche berücksichtigen. Zusätzlich kann ein solcher Detektor 3 am Objekt 4 (Fig. 2) selbst angebracht werden, so daß auch die Relativbewegungen zwischen dem Ultraschallaufnahmesystem 1 und dem Detektor 4 berücksichtigt werden können.

Fig. 2 zeigt die Darstellung zweier Bildteilbereiche eines Objekts 4, welches Lageartefakte 5, 5* aufweist. Diese Lageartefakte entstehen durch Relativbewegungen des Objekts 4 bzw. des Ultraschallaufnahmesystems 1 bzw. durch Relativbewegungen zwischen dem Ultraschallaufnahmesystem 1 und dem Objekt 4, die durch die Erfassung von periodischen Bewegungen des Objekts 4 nicht erfaßt werden.

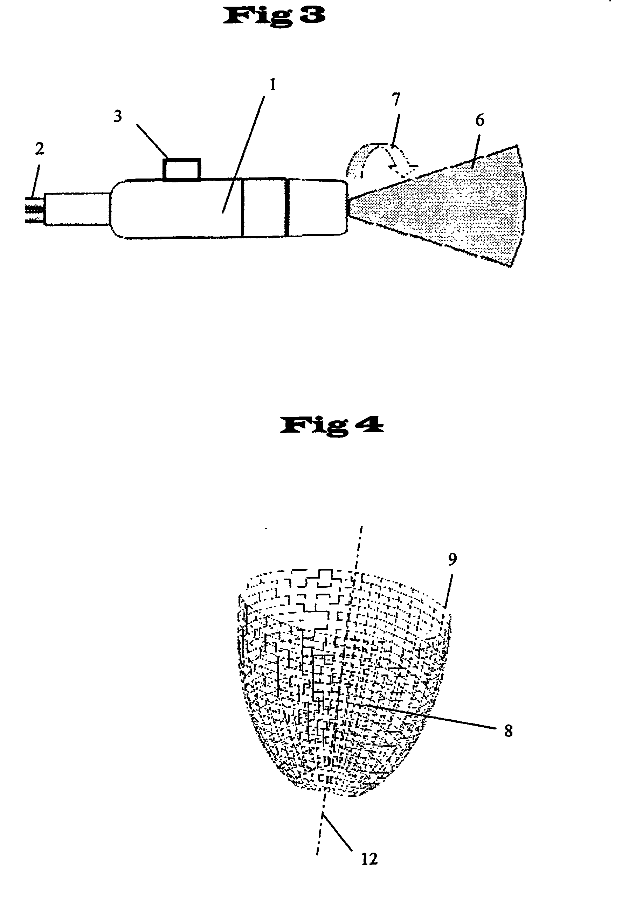

Fig. 3 zeigt wiederum ein Ultraschallaufnahmesystem 1 mit Detektor 3 und Datenleitung 2, wobei hier über die Datenleitung 2 sowohl die Daten der einzelnen Bildteilbereiche als auch die Daten des Detektors 3 an das Datenverarbeitungssystem (nicht dargestellt) übertragen werden. Das Ultraschallaufnahmesystem 1 ist hier ein Ultraschallkopf, der einen Ultraschallstrahl 6 längs einer rotatorischen Aufnahmerichtung 7 abstrahlt, um so das Objekt mittels des rotierenden Ultraschallstrahles 6 zu erfassen.

Der an der dreidimensionalen Ultraschallsonde, das heißt dem Ultraschallaufnahmesystem 1, angebrachte Detektor 3 kann beispielsweise ein Positionssensor sein, der die 6 Freiheitsgrade der Bewegung der Ultraschallsonde erfassen kann. Somit können "verwackelte" Bilder nachträglich anhand der Positionsdaten des Sensors einsortiert werden. Ebenfalls kann auch hier ein zusätzlicher Sensor am Patienten, beispielsweise am Brustbein des Patienten, angebracht werden, um evtl. Relativbewegungen des Patienten, die ebenfalls zu einer Relativbewegung des Organs, das heißt beispielsweise des Herzens führen, zu erfassen. Mittels der Auswertung der Positionsdaten des Sensors an der Ultraschallsonde und der Positionsdaten des Sensors am Patienten lassen sich dann die entsprechenden Bildteilbereiche bei der Darstellung des korrigierten Ultraschallbildes korrigieren.

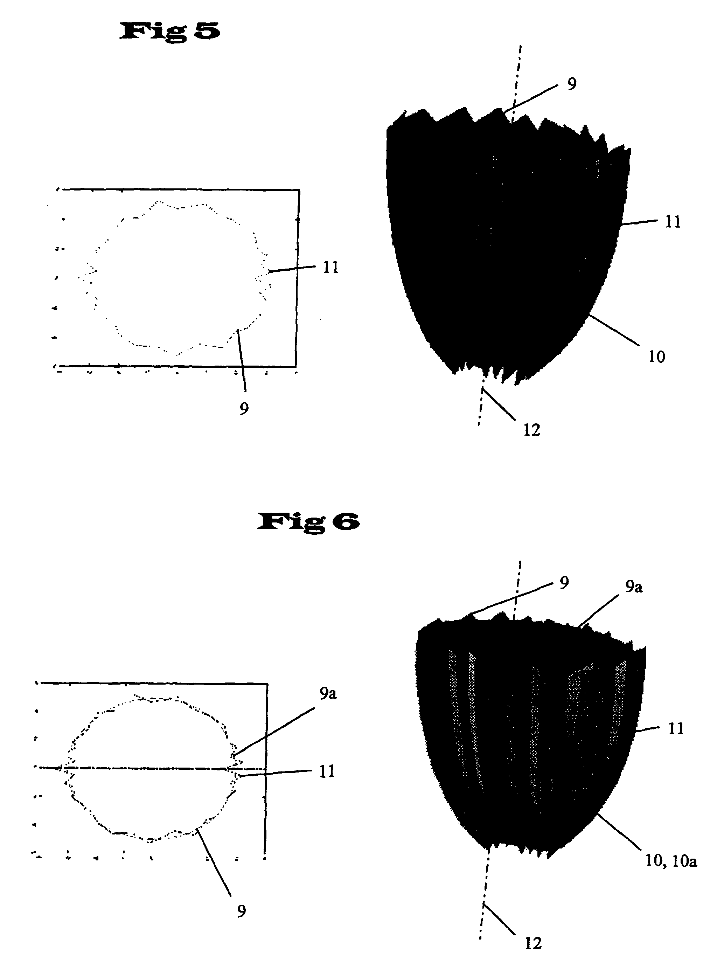

Die Fig. 4 bis 7 zeigen die Resultate eines weiteren vorteilhaften Ausführungsbeispiels der Erfindung. Hierbei werden die relativen Bewegungen zwischen dem Objekt 4 und dem Ultraschallaufnahmesystem 1 und/oder zufälligen oder auch periodischen Bewegungen des Objekts 4 mittels eines in Fig. 5 dargestellten Modellbildes 10 ermittelt und den einzelnen ursprünglich aufgenommenen Bildteilbereichen (Fig. 2) zur Erzeugung eines korrigierten Ultraschallbildes zugeordnet.

Dazu dient beispielsweise ein Korrekturalgorithmusverfahren, welches die bei tomographischen Aufnahmen auftretenden Bewegungsartefakte detektiert, die Lagefehler ermittelt und damit die Daten der einzelnen aufgenommenen Bildteilbereiche korrigiert, um dann zu einem korrigierten Ultraschallbild zu gelangen. Für die Bestimmung und Korrektur der Lagefehler, das heißt der durch die Bewegungsartefakte erzeugten Fehler der einzelnen Bildteilbereiche zueinander, wird vorteilhafterweise iterativ vorgegangen.

Zur Ermittlung der beschriebenen Bewegungen wird ein in Fig. 4 dargestelltes Oberflächenmodell 8 verwandt, welches einen oberen Rand 9 aufweist. Die Oberfläche des Oberflächenmodells 8 ist glatt, das heißt C²-stetig und krümmungsminimiert, wobei angenommen wird, daß die auftretenden Lageartefakte in allen sechs räumlichen Freiheitsgraden normalverteilt sind. Zu jeder einzelnen tomographischen Schnittebene, das heißt zu jedem einzelnen Bildteilbereich werden die Konturen des Oberflächenmodells 8, insbesondere die Oberfläche des Oberflächenmodells 8, als bekannt angenommen. Dies ist in Fig. 4 durch die oberste Schnittebene, d.h. durch den Rand 9 dargestellt. Das nachfolgend als Beispiel erläuterte Aufnahmeverfahren wird derart durchgeführt, daß die einzelnen Bildteilbereiche die z-Achse 12, wie in Fig. 4 dargestellt, als gemeinsame Rotationsachse besitzen:

In Fig. 4 ist das Oberflächenmodell 8 des aufzunehmenden Objekts 4 als Draht-Gitter-Modell dargestellt. Eine tomographische Abbildung dieses Objekts 4 mit dem oben beschriebenen Aufnahmeverfahren liefert in jeder Aufnahmeebene, das heißt in der entsprechenden Ebene der einzelnen Bildteilbereiche, eine unten offene Parabelkontur.

Durch die Bewegungsartefakte des Objekts 4 ergibt sich eine relative Bewegung des Koordinatensystems der Aufnahmeapparatur, das heißt des Ultraschallaufnahmesystems 1, gegenüber dem Koordinatensystem des Objekts 4 zwischen den einzelnen Aufnahmen statistisch in allen sechs räumlichen Freiheitsgraden, das heißt, daß sich entweder das Objekts 4 relativ zum Ultraschallaufnahmesystem 1 bzw. vice versa oder sich das Objekt 4 zufällig oder periodisch während der Aufnahme der einzelnen Bildteilbereiche bewegt, so daß die einzelnen Bildteilbereiche untereinander Lageartefakte aufweisen. Eine direkte Rekonstruktion, das heißt die Zusammensetzung der entsprechend aufgenommenen Bildteilbereiche führt dann zu dem in Fig. 5 dargestellten rekonstruierten Modellbild 10. Dieses rekonstruierte Modellbild 10 ist durch eine zackige rekonstruierte Kontur 11 gekennzeichnet, die sich besonders leicht durch den oberen Rand 9 des rekonstruierten Modellbildes 10 in einer Schnittdarstellung (links neben der dreidimensionalen Darstellung in Fig. 5 gezeigt) darstellen läßt.

Zur Ermittlung des korrigierten Ultraschallbildes, d.h. zur Korrektur der Daten der aufgenommenen Bildteilbereiche, läßt sich das in Fig. 5 dargestellte rekonstruierte Modellbild 10 approximieren. Hierzu dient das in Fig. 4 dargestellte Oberflächenmodell 8. Die relativen Abweichungen zwischen dem rekonstruierten und dem approximierten Modellbild (10a) werden ermittelt, um so die Bewegungsartefakte schätzen zu können.

Die relativen Abweichungen dienen zur Korrektur aller Daten der aufgenommenen Bildteilbereiche, während für die Approximation, die zur Ermittlung der relativen Abweichungen dient, lediglich Oberflächeninformationen herangezogen werden (bsp. durch das in Fig.4 dargestellte Oberflächenmodell 8).

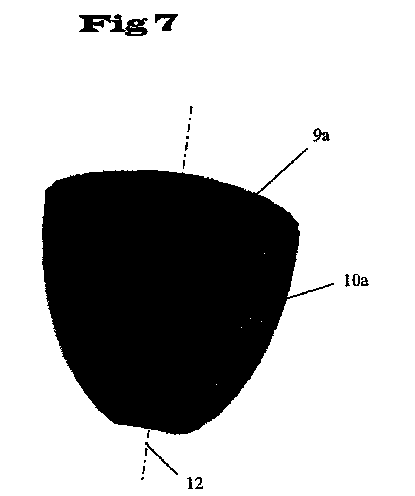

Eine Überlagerung des approximierten Modellbildes 10a und des rekonstruierten Modellbildes 10 zur Bestimmung der relativen Abweichungen zeigt Fig. 6. Diese Überlagerung läßt sich besonders deutlich durch die Darstellung des oberen Randes 9 des rekonstruierten Modellbildes 10, der mit dem oberen Rand 9a des approximierten Modellbildes 10a (in Fig. 6 links dargestellt) überlagert wird, darstellen. Die rekonstruierte Kontur 11, die noch die Bewegungsartefakte aufweist, ist ebenfalls in Fig. 6 dargestellt, wobei die rekonstruierte Kontur 11 im oberen Bereich des dreidimensionalen rekonstruierten Modellbildes 10 dem oberen Rand 9 entspricht. Durch die Approximation der Oberfläche des rekonstruierten Modellbildes lassen sich dann die relativen Abweichungen feststellen, aufgrund derer sich die aufgenommenen Bildteilbereiche, d.h. das Ultraschallbild mit allen räumlichen Daten, durch Schätzung korrigieren lassen.

Das approximierte Modellbild 10a kann mittels einer Minimierung des quadratischen Fehlers zwischen dem rekonstruierten Modellbild 10 und dem Oberflächenmodell 8 ermittelt werden. Gleichzeitig können auch bestimmte Glattheitsanforderungen berücksichtigt werden, die der Oberfläche des Oberflächenmodells 8 zugrunde gelegt wurden. Auf Basis dieses in Fig. 7 dargestellten approximierten Modellbildes 10a lassen sich dann die relativen Abweichungen der rekonstruierten Konturen 11 in jeder einzelnen Schnittebene, das heißt eines jeden einzelnen aufgenommenen Bildteilbereiches in allen sechs Freiheitsgraden durch Rechnung oder durch Schätzung ermitteln.

Nach einer entsprechenden Korrektur wird nun ein weiteres Modellbild 10a approximiert, wobei in Abhängigkeit des dabei erreichten Approximationsfehlers die Glattheitsanforderungen oder andere bestimmte Anforderungen an das Modellbild schrittweise reduziert werden können (Multiskalenansatz). Im Grenzfall geht so das approximierte Modellbild 10a in ein interpoliertes Modellbild 10a über. Dieses besitzt dann zwar keine regularisierenden Eigenschaften mehr, das heißt es können dabei keine Lagekorrekturen bzw. relative Abweichungen mehr berechnet bzw. geschätzt werden, jedoch stellt es eine bezüglich der Modellannahmen durch die Festlegung einer Modelloberfläche 8 optimale Schätzung des Oberflächenverlaufs zwischen den einzelnen Schnittebenen, das heißt den einzelnen korrigierten Bildteilbereichen, dar. Diese Informationen lassen sich dann gewinnbringend bei der Rekonstruktion eines signaltheoretisch unterabgetasteten Volumendatensatzes verwenden.

Die Schätzung der Lagefehler, das heißt der relativen Abweichungen, ist dabei optimal im Sinne der Annahmen des Oberflächenmodells 8. Das bedeutet, daß eine Approximation unter Minimierung des Fehlerquadrates bei normalverteilten Lagefehlern eine optimale Schätzung darstellt. Liegen jedoch andere Verteilungen der relativen Abweichungen zugrunde, muß das Approximationsverfahren entsprechend modifiziert werden.

## Patentansprüche

1. Verfahren zur Bewegungskompensation bei Ultraschallaufnahmen eines Objekts, wobei mittels eines Ultraschallaufnahmesystems einzelne Bild-Teilbereiche des Objekts aufgenommen werden,
mindestens ein Teil des Ultraschallaufnahmesystems zur Aufnahme der einzelnen Bild-Teilbereiche relativ zum Objekt verfahren und/oder zumindest teilweise rotiert wird, und
die einzelnen Bild-Teilbereiche zur Erzeugung eines Ultraschallbildes zusammengesetzt werden, wobei
a) Eigenbewegungen des Objekts (4) mittels mindestens eines Detektors (3) erfaßt und/oder
b) relative Bewegungen zwischen dem Objekt (4) und dem Ultraschallaufnahmesystem (1) und/oder Eigenbewegungen des Objekts (4) mittels eines vorgebbaren Modellbildes (10, 10a) des Objekts (4) ermittelt werden,
c) anhand der Erfassung bzw. Ermittlung der Eigenbewegungen des Objekts (4) und/oder der relativen Bewegungen zwischen dem Objekt (4) und dem Ultraschallaufnahmesystem (1) Positionsdaten des Objekts (4) und/oder des Ultraschallaufnahmesystems (1) ermittelbar sind,
d) die Positionsdaten den einzelnen Bild-Teilbereichen zur Erzeugung eines korrigierten Ultraschallbildes zugeordnet werden,
**dadurch gekennzeichnet, daß** das Modellbild (10, 10a) mittels eines vorgebbaren Drahtgittermodells (8) des Objekts (4) geglättet wird, um die durch die Bewegungen entstandenen Lageartefakte des Objekts (4) zu minimieren.

2. Verfahren nach Anspruch 1,
**dadurch gekennzeichnet,**
**daß** das Objekt (4) mit einem Detektor (3) verbunden wird, oder daß das Objekt (4) und das Ultraschallaufnahmesystem (1) mit Detektoren (3) verbunden werden, die die Bewegungen des Objekts (4) und/oder des Ultraschallaufnahmesystems (1) erfassen.

3. Verfahren nach Anspruch 1 oder 2,
**dadurch gekennzeichnet,**
**daß** durch den Detektor (3) drei translatorische und/oder drei rotatorische Freiheitsgrade der Bewegungen erfaßt werden.

4. Verfahren nach einem der vorhergehenden Ansprüche,
**dadurch gekennzeichnet,**
**daß** die Eigenbewegungen des Objekts (4) mittels des mindestens einen Detektors (3) während der Aufnahme der Bild-Teibereiche erfaßt werden.

5. Verfahren nach einem der vorhergehenden Ansprüche,
**dadurch gekennzeichnet,**
**daß** zur Ermittlung des korrigierten Ultraschallbildes ein Modellbild (10) anhand der einzelnen aufgenommenen Bild-Teilbereiche rekonstruiert wird,
**daß** das rekonstruierte Modellbild (10) approximiert bzw. geglättet wird,
**daß** relative Abweichungen zwischen dem rekonstruierten Modellbild (10) und dem approximierten Modellbild (10a) ermittelt werden, und
**daß** die relativen Abweichungen zur Ermittlung der Positionsdaten zur Erzeugung des korrigierten Ultraschallbildes verwendet werden.

6. Verfahren nach Anspruch 5,
**dadurch gekennzeichnet,**
**daß** die relativen Abweichungen in den drei translatorischen und den drei rotatorischen Freiheitsgraden ermittelt werden.

7. Verfahren nach einem der Ansprüche 5 oder 6,
**dadurch gekennzeichnet,**
**daß** zur Approximation eine für den statistischen Lagefehler zwischen dem rekonstruierten Modellbild (10) und einem Oberflächenmodell (8) geeignete Fehlernorm angewendet wird.

8. Verfahren nach einem der Ansprüche 5 - 7,
**dadurch gekennzeichnet,**
**daß** nach der Ermittlung der relativen Abweichungen zwischen dem rekonstruierten Modellbild (10) und dem approximierten Modellbild (10a) in Abhängigkeit des Approximationsfehlers sukzessive weitere Modellbilder approximiert werden.

9. Verfahren nach einem der vorhergehenden Ansprüche,
**dadurch gekennzeichnet,**
**daß** die Eigenbewegungen des Objekts (4) aus zufälligen und/oder periodischen Bewegungen des Objekts (4) bestehen.

10. Verfahren nach einem der vorhergehenden Ansprüche,
**dadurch gekennzeichnet,**
**daß** die Zeitpunkte zur Aufnahme von einzelnen Bild-Teilbereichen des Objekts (4) durch die erfaßten periodischen Bewegungen des Objekts (4) gesteuert bzw. geregelt werden.

11. Verfahren nach Anspruch 10 zur Aufnahme eines menschlichen oder tierischen Organs,
**dadurch gekennzeichnet,**
**daß** die Zeitpunkte zur Aufnahme von einzelnen Bild-Teilbereichen anhand von Signalen des Elektrokardiogramms, der Atmung, der Magenbewegung, der Perestaltik der Speiseröhre oder einer Kombination aus diesen Signalen des Lebewesens gesteuert bzw. geregelt werden.

12. Verfahren nach einem der vorhergehenden Ansprüche,
**dadurch gekennzeichnet,**
**daß** einzelne Bereiche des Objekts (4) bei der Darstellung farblich gekennzeichnet werden, insbesondere unterschiedlich bewegte Bereiche des Objekts (4).

13. Verfahren nach einem der vorhergehenden Ansprüche,
**dadurch gekennzeichnet,**
**daß** die korrigierten Ultraschallbilder, die aus den Bild-Teilbereichen gebildet werden, während der Aufnahme des Objekts (4) in Echtzeit verarbeitet und/oder angezeigt werden.

## Claims

1. Method of compensating motion in ultrasound images of an object, wherein at least one ultrasonic recording system is used to record separate partial image regions of an object,
at least one part of said ultrasonic recording system is displaced and/or rotated, at least partly, for recording said separate partial image regions, and
said separate partial image regions are composed to create an ultra sonogram, with
(a) the proper motions of said object (4) being detected by means of a detector (3) and/or
(b) relative motions between said object (4) and said ultrasonic recording system (1) and/or proper motions of said object (4) being determined by means of a predeterminable model image (10, 10a) of said object (4),
(c) position data of said object (4) and/or said ultrasonic recording system (1) being determinable on the basis of the detection or determination of the proper motions of said object (4) and/or the relative motions between said object (4) and said ultrasonic recording system (1),
(d) position data being associated with said separate partial image regions for creating a corrected ultra sonogram,
**characterised in**
**that** said model image (10, 10a) is smoothed by means of a predeterminable wire netting model (8) of said object (4) for minimising the positional artefacts of said object (4), which have been created by the motions.

2. Method according to Claim 1,
**characterised in**
**that** said object (1) is connected to a detector (3) or that said object (4) and said ultrasonic recording system (1) are connected to detectors (3) that detect the motions of said object (4) and/or said ultrasonic recording system.

3. Method according to Claim 1 or 2,
**characterised in**
**that** three translational and/or three rotational degrees of freedom of the motions are detected by said detector (3).

4. Method according to any of the preceding Claims,
**characterised in**
**that** the proper motions of said object (4) are detected by means of said at least one detector (3) during the recording of said partial image regions.

5. Method according to any of the preceding Claims,
**characterised in**
**that** a model image (10) is reconstructed on the basis of said partial image regions recorded for determining the corrected ultra sonogram,
**that** said reconstructed model image (10) is approximated or smoothed,
**that** relative variations between said reconstructed model image (10) and said approximated model image (10a) are determined, and
**that** the relative variations are used to determine the position data for creating the corrected ultra sonogram.

6. Method according to Claim 5,
**characterised in**
**that** said relative variations are determined in said three translational and said three rotational degrees of freedom.

7. Method according to any of the Claims 5 or 6,
**characterised in**
**that** for said approximation an error standard is applied that is suitable for the statistical positional error between said reconstructed model image (10) and a surface model (8).

8. Method according to any of the Claims 5 to 7,
**characterised in**
**that** after the determination of said relative variations between said reconstructed model image (10) and said approximated model image (10a) further model images are approximated in succession as a function of the approximation error.

9. Method according to any of the preceding Claims,
**characterised in**
**that** said proper motions of said object (4) consist of random and/or periodic motions of said object (4).

10. Method according to any of the preceding Claims,
**characterised in**
**that** the points of time for recording separate partial image regions of said object are controlled or feedback-controlled by the detected periodic motions of said object (4).

11. Method according to Claim 10 for recording a human or animal organ,
**characterised in**
**that** the points of time for recording separate partial image regions are controlled or feedback-controlled on the basis of signals from the electrocardiogram, from respiration, from the movement of the stomach, from peristalsis of the oesophagus or on the basis of a combination of these signals of the living organism.

12. Method according to any of the preceding Claims,
**characterised In**
**that** individual areas of said object (4) are identified by different colours in the representation, particularly areas of the object, which undergo different movements.

13. Method according to any of the preceding Claims,
**characterised in**
**that** said corrected ultra sonograms, which are created from said partial image regions, are processed and/or displayed in real time during the recording of said object (4).

## Revendications

1. Procédé à compenser le mouvement lors de prises d'images ultrasoniques d'un objet, dans lequel au moins un système de prise d'images ultrasoniques est utilisé afin d'enregistrer des plages partielles séparées de l'image d'un objet,
au moins une partie dudit système de prise d'images ultrasoniques est déplacée et/ou tournée, au moins en partie, pour l'enregistrement desdites plages partielles séparées de l'image, et
lesdites plages partielles séparées de l'image sont composées de façon à créer un ultrasonogram,
(a) aux mouvements propres dudit objet (4) étant détectés un détecteur (3) et/ou
(b) aux mouvements relatifs entre ledit objet (4) et ledit système de prise d'images ultrasoniques (1) et/ou mouvements propres dudit objet (4) étant déterminés moyennant une image type prédéterminable (10, 10a) dudit objet (4),
(c) aux données de position dudit objet (4) et/ou dudit système de prise d'images ultrasoniques (1) étant déterminables à la base de la détection ou la détermination des mouvements propres dudit objet (4) et/ou des mouvements relatifs entre ledit objet (4) et ledit système de prise d'images ultrasoniques (1),
(d) aux données de position étant affectées auxdits plages partielles séparées de l'image afin de créer un ultrasonogram corrigé,
**caractérisé en ce**
**que** ladite image type (10, 10a) est lissée moyennant un modèle en réseau de fils (8) dudit objet (4) afin de minimiser les artéfacts dudit objet (4), qui étaient crées par les mouvements.

2. Procédé selon la revendication 1,
**caractérisé en ce**
**que** ledit objet (1) est relié à un détecteur (3) ou en ce que ledit objet (4) et ledit système de prise d'images ultrasoniques (1) sont reliés aux détecteurs (3), qui détectent les mouvements dudit objet (4) et/ou dudit système de prise d'image ultrasoniques.

3. Procédé selon la revendication 1 ou 2,
**caractérisé en ce**
**que** trois degrés de liberté de translation et/ou de rotation des mouvements sont détectés par ledit détecteur (3).

4. Procédé selon une quelconque des revendications précédentes,
**caractérisé en ce**
**que** les mouvements propres dudit objet (4) sont détectés moyennant ledit au moins un détecteur (3) au cours de l'enregistrement desdites plages séparées de l'image.

5. Procédé selon une quelconque des revendications précédentes,
**caractérisé en ce**
**qu'**une image type (10) est reconstruite à la base de lesdites plages séparées de l'image enregistrée afin de déterminer ultrasonogram corrigé,
en ce que ladite image type reconstruite (10) est approchée par approximation ou lissée,
**que** des déviations relatives entre ladite image type reconstruite (10) et ladite image type approchée par approximation (10a) sont déterminées, et
**que** des déviations relatives sont utilisées afin de déterminer les données de position pour la création de ultrasonogram corrigé.

6. Procédé selon la revendication 5,
**caractérisé en ce**
**que** lesdites déviations relatives sont déterminées dans lesdits trois degrés de liberté de translation et lesdits trois degrés de liberté de rotation.

7. Procédé selon une quelconque des revendications 5 ou 6,
**caractérisé en ce**
**que** pour ladite approximation, une erreur standard est appliquée, qui se prête à l'erreur statistique de position entre ladite image type reconstruite (10) et un modèle surface (8).

8. Procédé selon une quelconque des revendications 1 à 7,
**caractérisé en ce**
**que**, suivant la détermination desdites déviations relatives entre ladite image type reconstruite (10) et ladite image type approchée par approximation(10a), des autres image types suivantes sont approchées par approximation en succession en fonction de l'erreur d'approximation.

9. Procédé selon une quelconque des revendications précédentes,
**caractérisé en ce**
**que** lesdits mouvements propres dudit objet (4) consistent en mouvements aléatoires et/ou périodiques dudit objet (4).

10. Procédé selon une quelconque des revendications précédentes,
**caractérisé en ce**
**que** les moments d'enregistrement de plages partielles séparées de l'image dudit objet sont commandés ou réglés par des mouvements périodiques dudit objet (4).

11. Procédé selon la revendication 10 pour l'enregistrement d'un organe humain ou animal,
**caractérisé en ce**
**que** les moments d'enregistrement de plages partielles séparées de l'image sont commandés ou réglés à la base de signaux, qui proviennent de l'électrocardiogramme, de la respiration, du mouvement de l'estomac, des contractions péristaltiques de l'oesophage ou à la base d'une combinaison de ces signaux de l'organisme vivant.

12. Procédé selon une quelconque des revendications précédentes,
**caractérisé en ce**
**que** des aires individuelles dudit objet (4) sont identifiées en couleurs différentes dans la représentation, en particulier des aires de l'objet, qui subissent des mouvements différents.

13. Procédé selon une quelconque des revendications précédentes,
**caractérisé en ce**
**que** les ultrasonogrammes corrigés, qui sont créés par dérivation desdites plages séparées de l'image, sont traités et/ou affichés en temps réel au cours de l'enregistrement dudit objet (4).
